# EUROPEAN PATENT APPLICATION

(11) **EP 4 706 637 A1**
(43) Date of publication of application: **11.03.2026**
(21) Application number: 25199123.8
(22) Date of filing: 29.08.2025
(51) Int. Cl.: A61K 9/127, A61K 9/51, A61K 31/7105, A61K 31/711, A61K 47/14, C12N 15/88, A61K 48/00, A61K 9/1272

(54) **DRUG FOR GENETIC MODIFICATION, DRUG DELIVERY METHOD, AND DRUG DELIVERY CARRIER**

(30) Priority: 29.08.2024 JP 2024147907
(71) Applicant: KABUSHIKI KAISHA TOSHIBA, Kawasaki-shi, Kanagawa (JP); The University of Tokyo, Bunkyo-ku, Tokyo 113-8654 (JP)
(72) Inventor: NOZAKI, Emi, Kawasaki-shi (JP); ISHIHARA, Mitsuko, Kawasaki-shi (JP); GOYAMA, Susumu, Tokyo, 113-8654 (JP); HIRAKI, Takamasa, Tokyo, 113-8654 (JP)
(74) Representative: Marks & Clerk LLP

(57) **Abstract**

According to one arrangement, a drug(1) for genetic modification according to an arrangement is a drug for performing genome editing on a gene in a hematopoietic stem cell. The drug for genetic modification contains a genome editing molecule(2) and a lipid nanoparticle(3) encapsulating the genome editing molecule. The lipid nanoparticle includes a lipid membrane(30) having a lumen. The lipid composition contains at least a first lipid (FFT-10) and a second lipid (FFT-20) in the lipid composition. The amount of the second lipid is larger than that of the first lipid, the total amount of the first lipid and the second lipid is 40 mol% or less, and the total amount of the cationic lipid is 60 mol% or less.

## Description

### FIELD

The present disclosure relates to a drug for genetic modification, a drug delivery method, and a drug delivery carrier.

### BACKGROUND

For example, myeloid leukemia is a disease caused by genetic abnormalities in hematopoietic stem cells. For the treatment, development of a method of correcting genomic abnormalities in hematopoietic stem cells using genome editing is expected.

For example, as a method of introducing a gene, a method using an electroporation method is known, but genome editing by the method needs to be performed ex vivo. For example, in a case where a gene is introduced into hematopoietic stem cells by an electroporation method, the gene is introduced into hematopoietic stem cells collected from a patient, and the normalized hematopoietic stem cells are retransplanted.

However, there is a concern that these processes impose a great burden on the patient. Therefore, there is a need to develop a technique that enables treatment in vivo with a less burden on the patient.

In addition, when genome editing is performed in hematopoietic stem cells, a safer and more efficient method for introduction is required regardless of in vivo or ex vivo.

### BRIEF DESCRIPTION OF THE DRAWINGS

FIG. 1 is a schematic diagram illustrating an example of a drug for genetic modification according to a first arrangement;
FIG. 2 is a schematic diagram illustrating an example according to the first arrangement;
FIG. 3 is schematic diagrams illustrating another example of the drug for genetic modification according to the first arrangement;
FIG. 4 is schematic diagrams illustrating another example of the drug for genetic modification according to the first arrangement;
FIG. 5 is a flowchart illustrating cleavage activity of a cleavage enzyme used in an example of an arrangement;
FIG. 6 is a schematic diagram illustrating an example of a drug for genetic modification according to a second arrangement;
FIG. 7 is a schematic diagram illustrating another example of the drug for genetic modification according to the second arrangement;
FIG. 8 is a flowchart illustrating an example of a method according to a third arrangement;
FIG. 9 is a schematic diagram illustrating an example of a kit according to a fourth arrangement;
FIG. 10 is a graph showing the results of Experiment 3;
FIG. 11 is a graph showing the results of Experiment 3;
FIG. 12 is a graph showing the results of Experiment 4; and
FIG. 13 is a graph showing the results of Experiment 6.

### DETAILED DESCRIPTION

According to one arrangement, a drug for genetic modification according to an arrangement is a drug for performing genome editing on a gene in a hematopoietic stem cell. The drug for genetic modification contains a genome editing molecule and a lipid nanoparticle encapsulating the genome editing molecule. The lipid nanoparticle includes a lipid membrane having a lumen. The lipid composition contains at least a first lipid (FFT-10) and a second lipid (FFT-20) in the lipid composition. The amount of the second lipid is larger than that of the first lipid, the total amount of the first lipid and the second lipid is 40 mol% or less, and the total amount of the cationic lipid is 60 mol% or less.

Hereinafter, arrangements will be described with reference to the accompanying drawings. Note that, in each of the arrangements, substantially the same constituents are denoted by the same reference numerals, and the description thereof may be partially omitted. The drawings are schematic, and a relationship between a thickness and a planar dimension of each part may be actually different from a ratio of the thickness of each part.

### (First Arrangement)

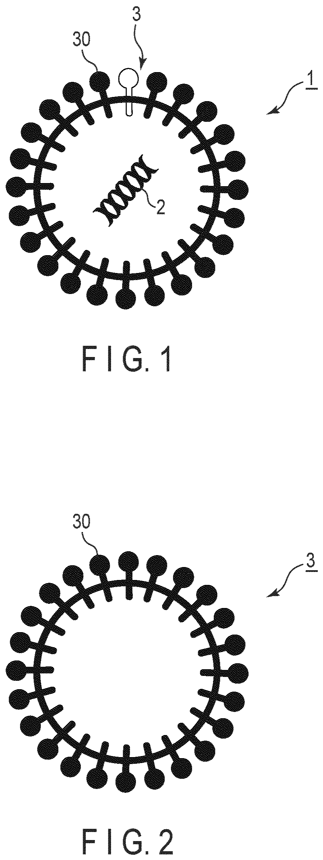

A drug for genetic modification (hereinafter, also simply referred to as "drug") according to a first arrangement will be described with reference to FIG. 1. A drug 1 for genetic modification contains a genome editing molecule 2 for performing genome editing on a hematopoietic stem cell, and a lipid nanoparticle 3 encapsulating the genome editing molecule 2.

The hematopoietic stem cell to which the drug for genetic modification is to be applied may be an isolated hematopoietic stem cell, may be an isolated hematopoietic stem cell in a state of coexisting with another cell, or may be a non-isolated hematopoietic stem cell. The non-isolated hematopoietic stem cell is, for example, a cell aggregate including hematopoietic stem cells such as peripheral blood, any tissue or fragment including hematopoietic stem cells, and a hematopoietic stem cell in a state of being contained in a living body. The tissue from which the hematopoietic stem cells are derived is, but is not limited to, tissue in which the hematopoietic stem cells can be present or a part thereof, such as bone marrow, cord blood, peripheral blood, liver, spleen, and hematopoietic tissue. If the drug for genetic modification is applied to the isolated hematopoietic stem cells in vitro, and then the treated cells are returned to the living body, that is, transplanted, it is desirable to use autologous cells.

An animal species to be a target for which the drug for genetic modification is to be used, that is, an animal species from which a hematopoietic stem cell to be subjected to genome editing is derived is not particularly limited. For example, such an animal species may be a mammal, including a human, or a non-human mammal. In other words, for example, the animal species may be an animal belonging to a primate such as a human or a monkey, a rodent such as a mouse, a rat, or a guinea pig, a companion animal such as a dog, a cat, or a rabbit, a livestock animal such as a horse, a cow, or a pig, or a display animal. That is, the drug for genetic modification may be used, for example, as a therapeutic agent for treating a disease in a human or an animal other than a human. In addition, the drug for genetic modification may be used as a reagent at a laboratory level for the purpose of academic or research and development of medicine or the like.

The lipid nanoparticle 3 is, for example, a substantially spherical hollow body formed of a lipid membrane 30 formed by arranging a plurality of lipid molecules as a material thereof in a non-covalent manner, that is, a lipid nanoparticle. The genome editing molecule 2 is encapsulated in the lumen formed at the center thereof. The lipid membrane 30 may be a lipid monomolecular membrane or a lipid bilayer membrane. In addition, the lipid membrane 30 may be formed of a single layer membrane or may be formed of a multi-layer membrane. The lipid membrane 30 is a sphere or a substantially sphere, and has a space capable of containing a substance in the vicinity of the center thereof. In other words, the lipid membrane 30 has a lumen.

The lipid membrane 30 contains, as constituent components thereof, at least FFT-10 that is a first lipid of Formula I and FFT-20 that is a second lipid of Formula II. In addition, the lipid membrane 30 contains the second lipid in a larger amount than the first lipid, and the total amount of the first lipid and the second lipid is 40% or less.

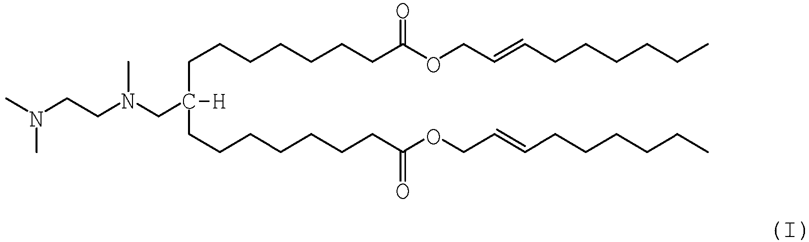

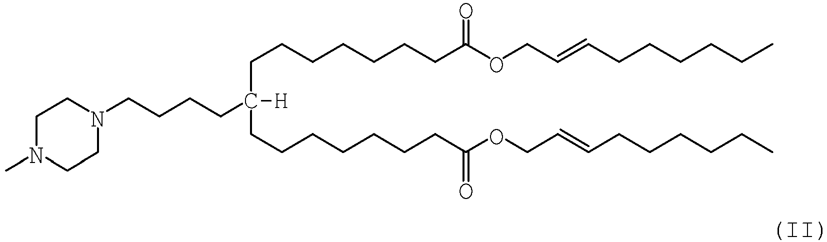

The lipid membrane 30 may contain an additional lipid in addition to the first lipid and the second lipid. Among the components constituting the lipid membrane 30, a fraction of the first lipid and the second lipid is hereinafter referred to as "first fraction". In addition, a fraction of a lipid molecular material other than the first lipid and the second lipid is hereinafter referred to as "second fraction".

The terms such as the first fraction and the second fraction represent a composition of the constituent components of the lipid membrane 30, and do not indicate the physical position of the lipid contained therein. For example, the components of the first fraction and the second fraction do not need to be integrated into an entity in the lipid membrane 30, and the lipid contained in the first fraction and the lipid contained in the second fraction can be present as a mixture.

In the present arrangement, a blending ratio of the first fraction with respect to the entire materials constituting the lipid membrane 30 is, for example, less than 40%, 30% to 40%, or 30% or more and less than 40%. In addition, the first lipid is 10% or less, for example, 5% or less, and more preferably 3% or less. In addition, the first lipid is 0.1% or more, and for example, it is desirable that the first lipid is 1% or more and further 2% or more. Herein, the percentage represents mol% unless otherwise specified.

For example, a blending ratio of the first lipid and the second lipid with respect to the entire materials constituting the lipid membrane 30 (the blending ratio of the first lipid : the blending ratio of the second lipid) can be 1 to 3:35 to 37, 1 to 3:32 to 34, 1 to 3:29 to 31, 3 to 7:30 to 33, 3 to 7:27 to 29, 7 to 10:28 to 30, 7 to 10:24 to 27, or 8 to 10:22 to 23. An upper limit and a lower limit of the range of the blending ratio herein may be adjusted so that the blending ratio of the first fraction with respect to the entire materials constituting the lipid membrane 30 described above is 40% or less. In other words, any upper limit value may be included, and any lower limit value may be included.

The type of lipid contained in the second fraction is not limited, and for example, the second fraction contains a base lipid. As the base lipid, for example, a lipid which is a main component of a biological membrane can be used. The base lipid is a phospholipid or a sphingolipid, for example, diacyl phosphatidylcholine, diacyl phosphatidylethanolamine, ceramide, sphingomyelin, dihydrosphingomyelin, kephalin, or cerebroside, or a combination thereof.

It is preferable to use, for example, as a base lipid,
1,2-dioleoyl-sn-glycero-3-phosphoethanolamine (DOPE),
1,2-stearoyl-sn-glycero-3-phosphoethanolamine (DSPE),
1,2-dipalmitoyl-sn-glycero-3-phosphatidylcholine (DPPC),
1-palmitoyl-2-oleoyl-sn-glycero-3-phosphatidylcholine (POPC),
1,2-di-O-octadecyl-3-trimethylammonium-propane (DOTMA),
1,2-dioleoyl-3-dimethylammonium-propane (DODAP),
1,2-dimyristoyl-3-dimethylammonium-propane (14:0 DAP),
1,2-dipalmitoyl-3-dimethylammonium-propane (16:0 DAP),
1,2-distearoyl-3-dimethylammonium-propane (18:0 DAP),
N-(4-carboxybenzyl)-N,N-dimethyl-2,3-bis(oleoyloxy)propane (DOBAQ),
1,2-dioleoyl-3-trimethylammonium-propane (DOTAP),
1,2-dioleoyl-sn-glycero-3-phosphocholine (DOPC),
1,2-dilinoleoyl-sn-glycero-3-phosphocholine (DLPC),
1,2-dioleoyl-sn-glycero-3-phospho-L-serine (DOPS), or
cholesterol, or any combination thereof.

It is also preferable that the second fraction contains a lipid which prevents aggregation of the lipid nanoparticles 3. Such a lipid is, for example, a PEG-modified lipid, in particular, polyethylene glycol dimyristoyl glycerol (DMG-PEG), maleimide-DMG-PEG, a polyamide oligomer derived from an omega-amino(oligoethylene glycol)alkanoic acid monomer (US 6,320,017 B), and a monosialoganglioside. The lipid that prevents aggregation of the lipid nanoparticles 3 is preferably contained in an amount of about 1% to about 5% (molar ratio) with respect to the entire lipids of the lipid membrane 30.

The second fraction may further contain a relatively less toxic lipid for modulating toxicity. Such a lipid is, for example, a lipid having a functional group that binds a ligand to the lipid nanoparticle 3 and a lipid such as a lipid sterol (for example, cholesterol) for suppressing leakage of the inclusion. In particular, the lipid nanoparticle 3 preferably contains cholesterol.

For example, when the second fraction contains DOPE, DOTAP, cholesterol, and DMG-PEG, the delivery efficiency of the genome editing molecule 2 is particularly excellent, which is preferable.

The lipid membrane 30 in the present arrangement contains 60% or less of a cationic lipid in its constituent components. For example, when the lipid membrane 30 contains the first lipid, the second lipid, DOPE and/or DOTAP, cholesterol, and DMG-PEG and/or maleimide-DMG-PEG, the first lipid, the second lipid, and DOTAP are 60% or less in total as a lipid composition (molar ratio). Hereinafter, the cationic lipid other than the first lipid and the second lipid is also referred to as "third lipid".

As described above, when the lipid membrane 30 of the lipid nanoparticle 3 contains the first lipid, the second lipid, DOPE and/or DOTAP, cholesterol, and DMG-PEG or maleimide-DMG-PEG, the nucleic acid encapsulation amount and the nucleic acid introduction efficiency are particularly excellent, which is preferable.

For example, the constituent components of the lipid membrane 30 include a neutral lipid, a third lipid, cholesterol, and a PEG-modified lipid, and the total thereof may be 70% or less. Desirably, the total thereof is 60% or more. For example, the lipid membrane 30 contains more third lipids than neutral lipids.

For example, the lipid membrane 30 has a lipid composition ratio of first lipid : second lipid : neutral lipid : third lipid : cholesterol : PEG-modified lipid = 1 to 3:29 to 31:1 to 3:16 to 18:44 to 46:3 to 5(mol%, 100 mol% in total).

As the base lipid, it is particularly preferable to use the third lipid or the neutral lipid, and an acid dissociation constant of the lipid nanoparticle can be adjusted depending on the content thereof. DOTAP is preferably used as the third lipid, and DOPE is preferably used as the neutral lipid. In addition, as described above, the total amount of the cationic lipid including the first lipid and the second lipid is 60% or less as a blending ratio with respect to the entire materials constituting the lipid membrane 30.

When the lipid nanoparticle 3 comes into contact with a hematopoietic stem cell, the lipid nanoparticle is taken up into the cell, for example, by endocytosis. As a result, the inclusion of the lipid nanoparticle 3 is released into the cell.

Basically, the lipid nanoparticle 3 contains the first lipid and the second lipid in the lipid membrane 30 and has a blending ratio of the constituent components as described above, thereby achieving a property that the lipid nanoparticle 3 is easily taken up by a hematopoietic stem cell and is hardly taken up by other cells such as a normal blood cell. According to a drug delivery carrier of an arrangement, it is possible to introduce the genome editing molecule 2 and the like to be delivered into a hematopoietic stem cell without using an antibody, a receptor, or the like. For example, the genome editing molecule 2 is contained inside the lipid nanoparticle 3, such that the genome editing molecule 2 can be introduced into a hematopoietic stem cell by, for example, a simple procedure of bringing the drug 1 into contact with the hematopoietic stem cell.

Therefore, genome editing performed by introducing a genome editing molecule into a hematopoietic stem cell can also be performed in vivo. In addition, since the lipid nanoparticle 3 is used, genome editing performed by introducing a genome editing molecule into a hematopoietic stem cell can be performed more safely than, for example, a method using a viral vector. Furthermore, the introduction efficiency of the genome editing molecule is higher than that of a method such as lipofection.

As described above, the lipid nanoparticle 3 has a property of being easily taken up by a hematopoietic stem cell and not easily taken up by other cells such as a normal blood cell. Thus, such a lipid nanoparticle 3 may be provided as a drug delivery carrier (FIG. 2). In this case, the lipid nanoparticle 3 may encapsulate any substance to be delivered to the hematopoietic stem cell as desired.

The genome editing molecule 2 is a molecule that edits a gene in a hematopoietic stem cell when introduced into the hematopoietic stem cell. For example, the genome editing molecule 2 may be any molecule that brings about DNA cleavage, binding, insertion, repair, and the like.

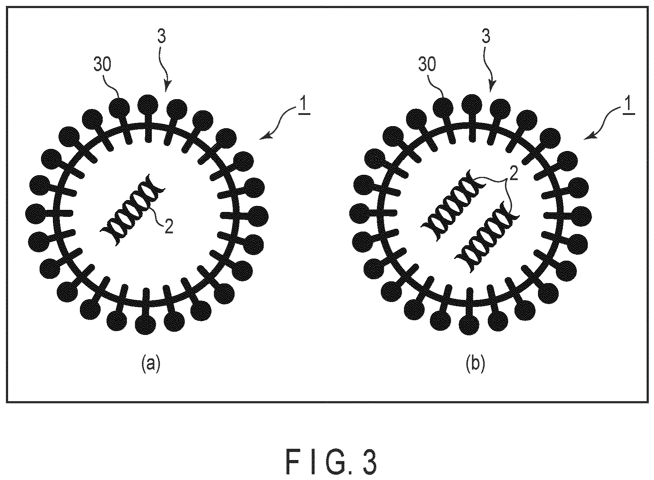

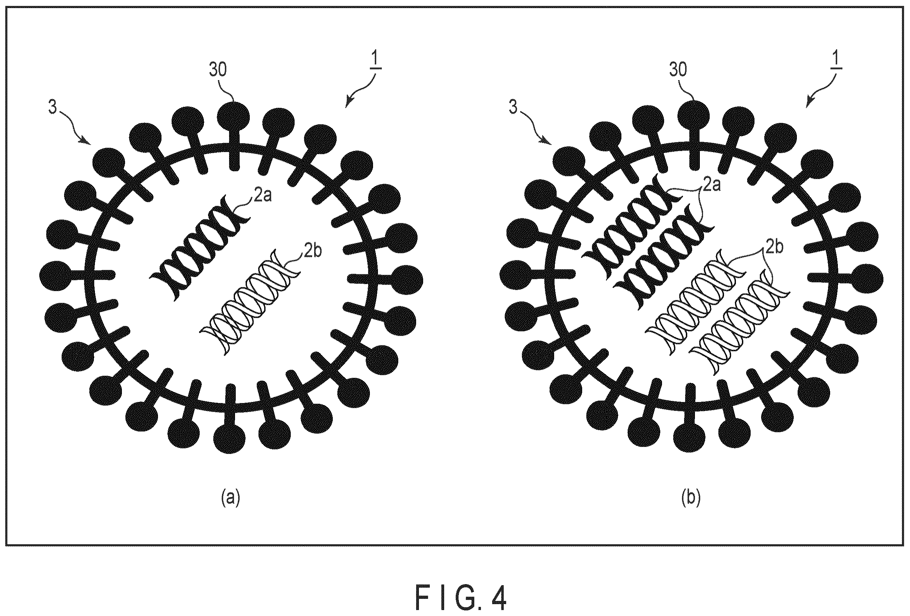

The drug 1 for genetic modification will be further described with reference to FIGS. 3 and 4. For example, the drug 1 for genetic modification may contain a single type of genome editing molecule 2 in a lipid nanoparticle 3 (part (a) of FIG. 3). Alternatively, the drug 1 for genetic modification may contain a plurality of genome editing molecules 2 in the same type in a lipid nanoparticle 3 (part (b) of FIG. 3). Alternatively, the drug 1 for genetic modification may contain each of two types of genome editing molecules 2 (part (a) of FIG. 4), may contain a plurality of two types of genome editing molecules 2 (part (b) of FIG. 4), or may contain each of three or more types of genome editing molecules 2 or a plurality of three or more types of genome editing molecules 2 (not illustrated), in a lipid nanoparticle 3. When a plurality of types of the genome editing molecules 2 are contained, the numbers thereof may be equal to or different from each other.

For example, the genome editing molecule 2 is a molecule having a function or a part of a function of losing a function of a target gene. The genome editing molecule 2 is, for example, a molecule having a function of cleaving a nucleic acid, a molecule that binds to a specific position of a target nucleic acid, a molecule that determines a cleavage site of a nucleic acid, and/or a molecule to be newly inserted into a target genome.

Such a genome editing molecule 2 is a genome editing enzyme capable of sequence-specific cleavage, for example, Cas protein. The Cas protein is, for example, CRISPR/Cas9, CRISPR/Cpf1, an analog derived from another bacterium of CRISPR/Cas system (SaCas9, ScCas9, FnCpf1, or the like), or another Cas protein (Cas12a, Cas12b, C2c1, C2c2, C2c3, or the like) and a modification thereof.

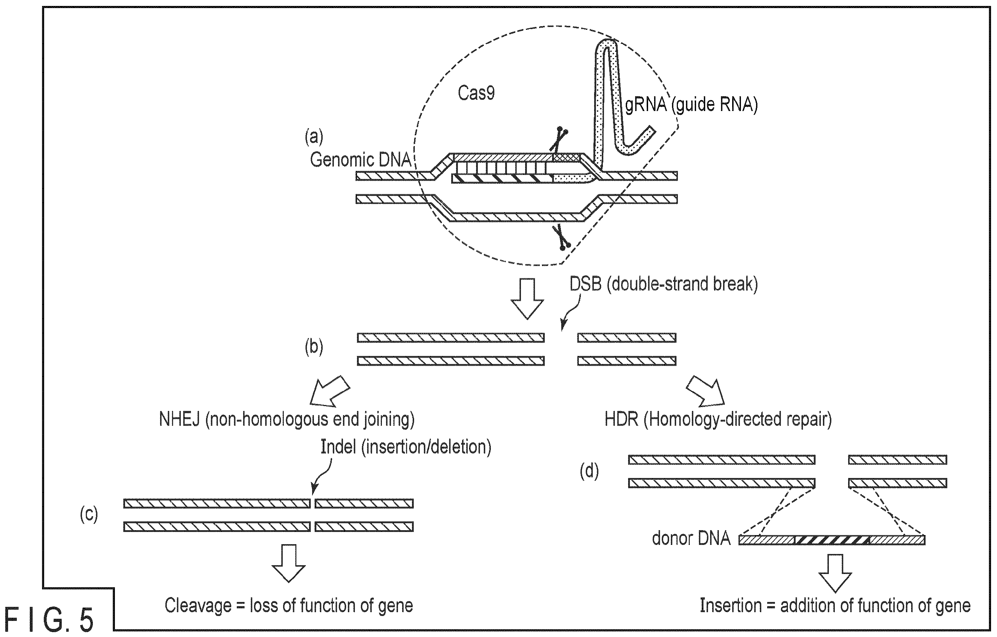

Here, CRISPR/Cas9 is also referred to as CRISPR-Cas9, for example. An outline of the CRISPR/Cas9 system will be described with reference to FIG. 5. Cas9 is a double-stranded DNA-cleaving enzyme. gRNA is guide RNA and is RNA containing a sequence complementary to a target gene. The guide RNA is responsible for moving Cas9 to a target site. Cas9 acts on a target sequence of genomic DNA to which the guide RNA is bound and cleaves the genomic DNA at a specific cleavage site (part (a) of FIG. 5). A double-strand break (DSB) is formed at the cleaved site (part (b) of FIG. 5). For example, when a specific gene is knocked out, a target gene may be cleaved and eliminated from genomic DNA by non-homologous end joining (NHEJ) (part (c) of FIG. 5). For example, when a desired gene is newly added to the genomic DNA, a previously prepared donor DNA may be incorporated into DSB by homology-directed repair (HDR) (part (d) of FIG. 5).

When the genome editing enzyme is Cas protein, the genome editing molecule 2 may further contain guide RNA in addition to the genome editing enzyme.

The guide RNA may be RNA that contains a guide sequence complementary to a target nucleic acid and has a function of guiding and specifically binding the genome editing enzyme to the target nucleic acid. The structure of the guide RNA is not particularly limited as long as it contains the guide sequence and has the above function.

For example, the guide RNA may be crRNA or a dual RNA of crRNA and tracrRNA. In addition, the guide RNA may be a single-stranded guide RNA (sgRNA) in which crRNA and tracrRNA are linked by a linker. The length of the guide RNA is, for example, from about 40 to about 150 bases.

For example, the crRNA contains a guide sequence of about 16 bases to 24 bases in length complementary to the target nucleic acid and a repeat region, the tracrRNA has an anti-repeat region complementary to the repeat region, and the crRNA and the tracrRNA form a double strand.

The guide RNA can contain about 1 molecule to about 1,000 molecules within the lipid nanoparticle.

The genome editing enzyme and the guide RNA may be encapsulated in the lipid nanoparticle as a nucleic acid encoding them. The nucleic acid may be DNA or RNA. In addition, the nucleic acid encapsulated in the lipid nanoparticle 3 may be a single-stranded nucleic acid, a double-stranded nucleic acid, a linear nucleic acid, or a cyclic nucleic acid.

The genome editing molecule 2 may further contain a donor nucleic acid, a compound that improves genome editing efficiency, and the like.

The donor nucleic acid is, for example, double-stranded DNA, and may be linear or circular. The length of the donor nucleic acid is, for example, about 3 bases to about 20,000 bases.

The sequence contained in the donor nucleic acid is a sequence to be introduced into the genome of the hematopoietic stem cell. For example, such a sequence is a gene expression set containing a promoter sequence, a specific gene, and a transcription termination sequence, a base sequence encoding a specific gene or a part of the gene, a natural base sequence that is not a gene, a non-natural base sequence, or the like. Alternatively, the sequence may be a base sequence encoding one to several amino acids, a sequence containing three to several tens of nucleotides, or the like.

The donor nucleic acid may contain, for example, another sequence in addition to the above sequences. Such a sequence may be a recognition sequence of a genome editing enzyme, a recognition sequence of guide RNA, or the like.

The donor nucleic acid may be selected according to the application of the lipid nanoparticle 3, and may be contained, for example, in about 1 molecule to about 100 molecules within the lipid nanoparticle.

The compound that improves or promotes genome editing efficiency may be, for example, a histone demethylase inhibitor.

In addition to the above, the genome editing molecule 2 may contain additional RNA. The additional RNA is, for example, RNA having a function of modifying DNA, such as methylation, demethylation, repair, and/or binding of DNA. For example, the RNA is RNA encoding a protein having the modifying activity. By containing such RNA, it is possible to add the modification to the sequence of the nucleic acid introduced into the genome and the sequence around the nucleic acid. Therefore, for example, a functional modification can be further added to the cell.

When the nucleic acid contained in the genome editing molecule 2 is RNA, the RNA is preferably modified to have degradation resistance. For example, the modification may be a known modification that prevents RNA from being degraded by RNase present inside and outside the cell. Such a modification is, for example, use/introduction of a naturally modified nucleotide or a non-natural nucleotide into RNA, use/addition of a non-natural sequence, addition of a natural/non-natural CAP structure, and the like.

Examples of the naturally modified nucleotide include pseudouridine, 5-methylcytidine, and 1-methyladenosine. The non-natural nucleotide is, for example, bridged nucleic acid (BNA), locked nucleic acid (LNA), peptide nucleic acid (PNA), or the like.

The non-natural sequence may be, for example, any artificially produced non-naturally occurring base sequence. The non-natural sequence is, for example, a random base sequence, a hybrid sequence of a natural/non-natural amino acid and a nucleic acid, or the like. The non-natural sequence is preferably added, for example, to the end of the RNA.

The natural CAP structure is, for example, CAPO (m7GpppN), CAP1 (m7GpppNm), or the like. The non-natural CAP structure is, for example, anti-reverse cap analog (ARCA), LNA-guanosine, or the like. The non-natural CAP structure is preferably added, for example, to the 5' end of the RNA.

The nucleic acid encapsulated in the lipid nanoparticle 3 may be enclosed in the lipid nanoparticle 3 in a state of being condensed with a nucleic acid condensing peptide. The nucleic acid condensing peptide is a peptide having a function of condensing a nucleic acid into a small size. By using the nucleic acid condensing peptide, a large amount of nucleic acid can be enclosed in the lipid nanoparticle, and the particle size of the lipid nanoparticle can be reduced. In addition, the amount of nucleic acids remaining outside the lipid nanoparticle is reduced, thereby preventing aggregation of the lipid nanoparticles. As a result, the delivery efficiency of the nucleic acid can be improved.

A preferred example of the nucleic acid condensing peptide is, for example, a peptide containing a cationic amino acid in 45% or more of the total. In a more preferred example of the nucleic acid condensing peptide, RRRRRR (first amino acid sequence) is contained at one end of the amino acid sequence, and the sequence RQRQR (second amino acid sequence) is contained at the other end. No or one or more intermediate sequences containing RRRRRR or RQRQR are contained between both amino acid sequences. In addition, two or more neutral amino acids are contained between two adjacent sequences among the first amino acid sequence, the second amino acid sequence, and the intermediate sequence. The neutral amino acid may be, for example, G or Y.

The nucleic acid condensing peptide preferably contains the following amino acid sequences:
· RQRQRYYRQRQRGGRRRRRR (SEQ ID NO: 1); and
· RQRQRGGRRRRRR (SEQ ID NO: 2).

According to such a nucleic acid condensing peptide, it is possible to efficiently condense the nucleic acid by cationic arginine and to weaken the anionicity of the nucleic acid. Accordingly, it is possible to efficiently enclose the nucleic acid in the lipid nanoparticle 3. Furthermore, since the nucleic acid condensing peptide efficiently dissociates the nucleic acid in the cell, it is possible to efficiently express the nucleic acid introduced into the hematopoietic stem cell.

Alternatively, the nucleic acid condensing peptide contains RRRRRR (third amino acid sequence) at one end and RRRRRR (fourth amino acid sequence) at the other end. No or one or more intermediate sequences containing RRRRRR or RQRQR are contained between both amino acid sequences. In addition, two or more neutral amino acids can be contained between two adjacent sequences among the third amino acid sequence, the fourth amino acid sequence, and the intermediate sequence.

Such a nucleic acid condensing peptide preferably contains the following amino acid sequence:
· RRRRRRYYRQRQRGGRRRRRR (SEQ ID NO: 3).

Such a nucleic acid condensing peptide has strong cationicity at both ends and high binding to a nucleic acid. Therefore, it is possible to more efficiently condense the nucleic acid and encapsulate a larger amount of nucleic acids in the lipid nanoparticle 3. As a result, the amount of nucleic acids remaining outside the lipid nanoparticle 3 is reduced, thereby preventing aggregation of the lipid nanoparticles 3, making it easier for the lipid nanoparticle 3 to be taken up into a hematopoietic stem cell.

Furthermore, it is also possible to use a nucleic acid condensing peptide having the following amino acid sequence in combination with any of the nucleic acid condensing peptides.

For example, a peptide represented by the following amino acid sequence
· GNQSSNFGPMKGGNFGGRSSGPYGGGGQYFAKPRNQGGY(M9) (SEQ ID NO: 4)
can further condense a nucleic acid aggregate condensed with the nucleic acid condensing peptide. Therefore, it is possible to obtain a lipid nanoparticle having a smaller particle size. As the particle size is smaller, the lipid nanoparticle is more easily taken up into a hematopoietic stem cell, such that a nucleic acid can be more efficiently introduced into the hematopoietic stem cell. For example, the nucleic acid may be condensed by stirring and mixing the nucleic acid with the nucleic acid condensing peptide before being encapsulated in the lipid nanoparticle 3.

It is more preferable to use a nucleic acid condensing peptide from the viewpoint of achieving the effects described above. Note that, depending on the type of nucleic acid to be used, the culture conditions of the cells, or the like, selection may be made not to use the nucleic acid condensing peptide, and such an aspect is also included in the present arrangement.

The lipid nanoparticle 3 may encapsulate additional components as necessary. The additional components are, for example, a pH adjusting agent, an osmotic pressure adjusting agent, and the like. The pH adjusting agent is, for example, an organic acid such as citric acid and a salt thereof. The osmotic pressure adjusting agent is, for example, a sugar or an amino acid.

The lipid nanoparticle 3 encapsulating the genome editing molecule 2 and other components as necessary is produced, for example, by a known method used when a small molecule is enclosed in the lipid nanoparticle 3. Such a method is, for example, Bangham method, an organic solvent extraction method, a surfactant removal method, a freeze-thaw method, or the like. For example, in the case of the organic solvent extraction method, for example, first, a mixture in which the material of the lipid nanoparticle 3 is contained in an organic solvent such as alcohol is obtained. An aqueous buffer containing the components to be encapsulated is added thereto. By stirring and suspending the mixture, the lipid nanoparticles 3 encapsulating a desired component are produced.

The drug 1 for genetic modification according to the first arrangement may be administered to a target hematopoietic stem cell or a living body to be applied as it is or as a pharmaceutical composition mixed with an appropriate carrier or additive. The living body to be applied is, for example, an animal.

As described above, the drug 1 for genetic modification according to the first arrangement provides a technique for efficiently introducing a gene into a hematopoietic stem cell. In addition, according to the first arrangement, a drug delivery carrier having tropism for a hematopoietic stem cell (lipid nanoparticle having tropism for a hematopoietic stem cell) is provided. In addition, it is possible to correct or normalize gene abnormality in the hematopoietic stem cells in vivo by administering the drug 1 for genetic modification according to the first arrangement to a subject. According to the first arrangement, it is possible to efficiently introduce a gene into a hematopoietic stem cell. Such tropism to a hematopoietic stem cell enables, for example administration to a subject such as a patient directly as a medicine. For example, a treatment method that eliminates a need for bone marrow transplantation and reduces a burden on a subject such as a patient can be provided.

### (Second Arrangement)

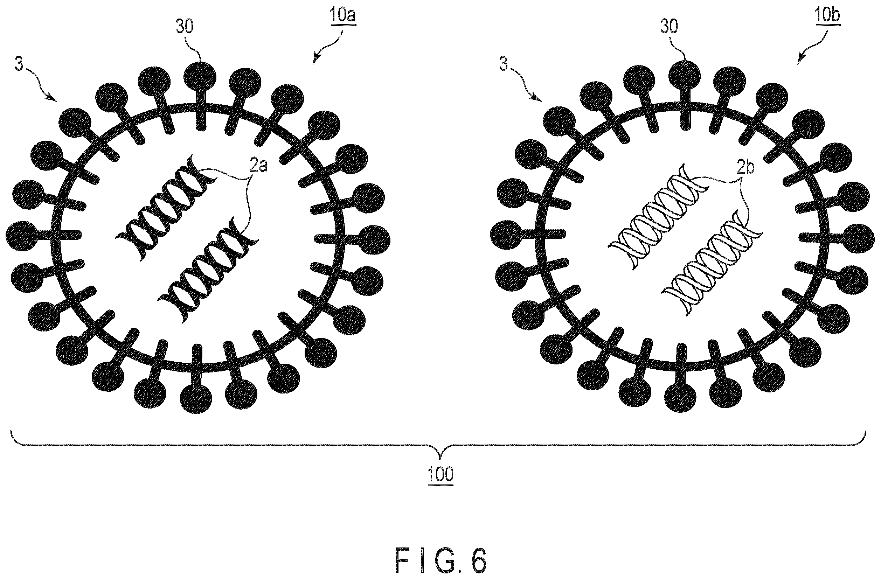

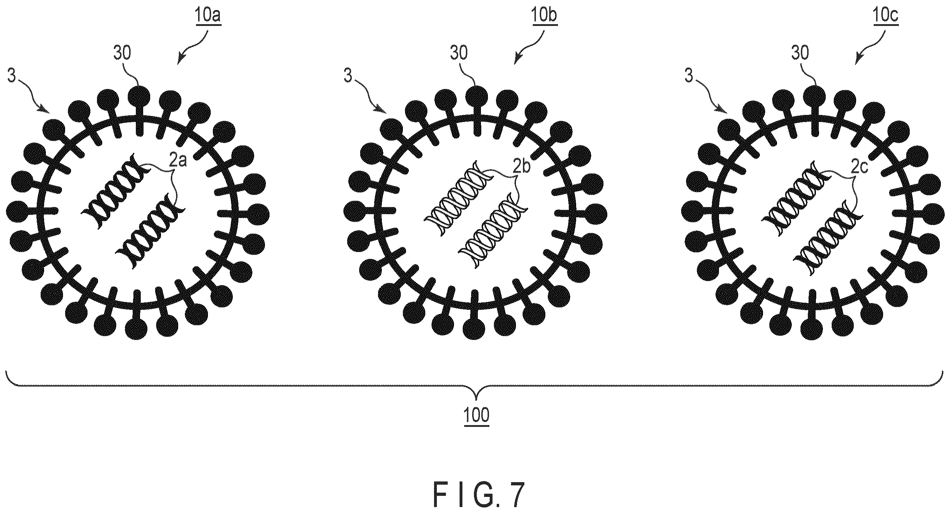

A second arrangement will be described with reference to FIGS. 6 and 7. As described above, the drug 1 for genetic modification according to the first arrangement may contain a plurality of types of genome editing molecules 2 as an inclusion of a lipid nanoparticle 3 (FIG. 4). In the second arrangement, an example in which a plurality of types of genome editing molecules 2 are contained in a lipid nanoparticle 3 for each type is illustrated.

A drug 100 for genetic modification illustrated in FIG. 6 contains a first component particle 10a and a second component particle 10b. The first component particle 10a includes a lipid nanoparticle 3 including a lipid membrane 30 and a first genome editing molecule 2a encapsulated in the lipid nanoparticle 3. The second component particle 10b includes a lipid nanoparticle 3 including a lipid membrane 30 and a second genome editing molecule 2b encapsulated in the lipid nanoparticle 3. It is also understood that the drug 100 for genetic modification is obtained by dividing the drug 1 for genetic modification in part (b) of FIG. 4 by the type of the genome editing molecules 2a and 2b, and encapsulating the drug 1 for genetic modification in the lipid nanoparticle 3.

The configurations of the lipid membranes 30 of the first component particle 10a and the second component particle 10b may be the same as or different from each other.

For example, as illustrated in FIG. 7, the drug 100 for genetic modification contains a first component particle 10a, a second component particle 10b, and a third component particle 10c. The first component particle 10a is a lipid nanoparticle 3 encapsulating a first genome editing molecule 2a. The second component particle 10b is a lipid nanoparticle 3 encapsulating a second genome editing molecule 2b. The third component particle 10c is a lipid nanoparticle 3 encapsulating a third genome editing molecule 2c. The first to third genome editing molecules 2a, 2b, and 2c are genome editing molecules different from each other.

Note that the drug 100 for genetic modification may contain a lipid nanoparticle 3 encapsulating two or more types or three or more types of genome editing molecules for each type. For example, the drug 100 for genetic modification may contain the lipid nanoparticles 3 each encapsulating a mixture of a plurality of types of genome editing molecules in combination of components that are partially or completely different from each other, or in combination of components that are partially or completely the same as each other, and in combination ratios that are partially or completely different from each other. In addition, the component particle 10a contained in the drug 100 for genetic modification may contain only a single type of genome editing molecule, may contain only a mixture of a plurality of types of genome editing molecules, or may be a mixture thereof. For example, the drug 100 for genetic modification may contain a component particle containing a single type of genome editing molecule and a component particle containing a mixture of a plurality of types of genome editing molecules.

For example, in the drug 100 for genetic modification illustrated in FIG. 6, the first genome editing molecule 2a may be a genome editing enzyme, and the second genome editing molecule 2b may be guide RNA. Alternatively, the first genome editing molecule 2a may contain a genome editing enzyme, and the second genome editing molecule 2b may be mixed with additional components (a singly type of a plurality of types) so as to contain guide RNA.

In the drug 100 for genetic modification illustrated in FIG. 6 or 7, for example, when a genome editing molecule contains a donor nucleic acid, for example, the donor nucleic acid may be contained in the first genome editing molecule 2a or the second genome editing molecule 2b, or may be encapsulated in a third lipid nanoparticle 3c as the third genome editing molecule 2c alone or in a state of being mixed with other components.

As described above, the drug 100 for genetic modification according to the second arrangement provides a technique for efficiently introducing a gene into a hematopoietic stem cell. It is possible to correct or normalize gene abnormality in the hematopoietic stem cells in vivo by administering the drug 100 for genetic modification according to the second arrangement to a subject. In addition, the drug 100 for genetic modification according to the second arrangement facilitates adjustment of the delivery amount of the genome editing molecule to the hematopoietic stem cell.

### (Third Arrangement)

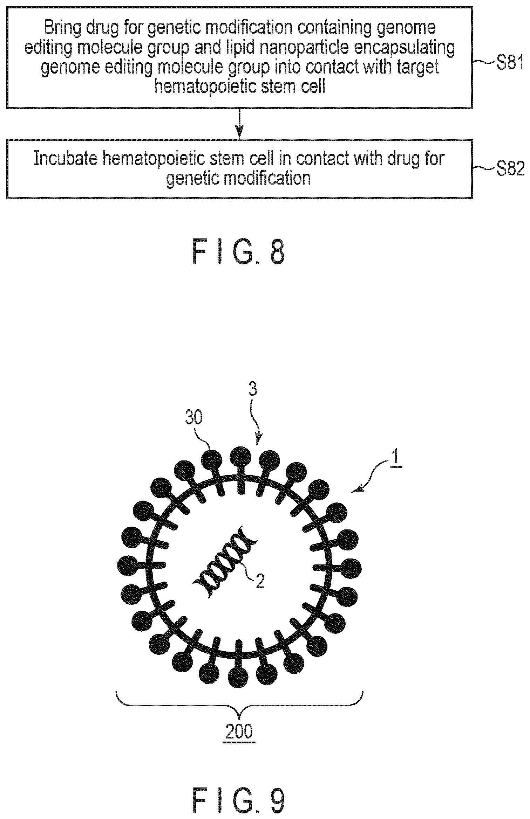

A third arrangement will be described with reference to FIG. 8. According to the third arrangement, a method of delivering a genome editing molecule 2 to a hematopoietic stem cell is provided. The method may also be referred to as a method of modifying a gene in a hematopoietic stem cell. The method includes bringing a drug 1 for genetic modification containing a genome editing molecule 2 and a lipid nanoparticle 3 encapsulating the genome editing molecule 2 into contact with a target hematopoietic stem cell (S81), and incubating the hematopoietic stem cell in contact with the drug 1 for genetic modification (S82).

The "target hematopoietic stem cell" may be any of the hematopoietic stem cells described above, any of the collected cell groups that may contain hematopoietic stem cells, an isolated or separated cell group, a collected tissue sample, a culture of extracted tissue, cultured cells, tissue, or the like, a mixture of any of them, or the like. In this case, the method may include preparing a target hematopoietic stem cell prior to S81. Such preparation may include, for example, obtaining, by the practitioner, a target hematopoietic stem cell collected, isolated, or extracted from a living body or a culture. In addition, the contact between the drug for genetic modification and the target hematopoietic stem cell is performed in vitro. In other words, the method can also be a genetic modification method of a hematopoietic stem cell including S81 and S82. Alternatively, the method can also be referred to as a method of editing genome of a hematopoietic stem cell, or a method of normalizing a hematopoietic stem cell having some defects or abnormality.

When the method is performed in vitro for the purpose of transplanting a hematopoietic stem cell to which the genome editing molecule 2 is delivered into a living body, it is desirable to prepare a target hematopoietic stem cell derived from the same species in advance. Derived from the same species means autologous or donor-derived. For example, it is also preferable to prepare an autologous hematopoietic stem cell.

In S81, the lipid nanoparticle 3 is brought into contact with the hematopoietic stem cell. When the hematopoietic stem cell is a cell removed ex vivo, contact may include, for example, suspending the cell in a medium, a buffer, saline, or the like, and adding the lipid nanoparticle 3 to the suspension. Alternatively, the contact may be or include adding the lipid nanoparticles 3 to a sample containing cells. The method may further include performing gentle stirring after adding the lipid nanoparticles 3.

The contact between the lipid nanoparticle 3 encapsulating the genome editing molecule 2 and the blood cell group may be performed at least once, or may be performed two or more times. In a case where the contact is performed a plurality of times, the interval of each contact may be, for example, every 6 hours, every 12 hours, every 24 hours, every 36 hours, every 48 hours, or a combination thereof.

When there are a plurality of types of the genome editing molecules 2 to be introduced, the genome editing molecules 2 may be encapsulated in a lipid nanoparticle 3, or may be encapsulated in a lipid nanoparticle as a mixture of each type or a desired formulation in the plurality of lipid nanoparticles 3. In addition, the lipid nanoparticle 3 may be brought into contact with the hematopoietic stem cell simultaneously, sequentially, time-dependent, continuously, and/or intermittently. For example, the contact may be accomplished by simultaneous addition or a plurality of times of addition, for example, sequential, time-dependent, continuously, and/or intermittent addition. Alternatively, the hematopoietic stem cells may be added to a medium or a buffer containing the drug 1 for genetic modification according to the arrangement.

The hematopoietic stem cells are incubated after contact with the lipid nanoparticles 3 encapsulating the genome editing molecule 2 (S82). The incubation may be accomplished, for example, by being maintained under certain conditions. The specific conditions may be conditions appropriate for maintaining the hematopoietic stem cells. In addition, for example, the incubation may be culture, and in this case, may be maintained under known culture conditions. The culture conditions may be, for example, known conditions suitable for hematopoietic stem cells. Such culture conditions may be, for example, in any medium (for example, a medium obtained by adding cytokines such as SCF, TPO, and FLT3L to StemSpan (registered trademark), Iscove's Modified Dulbecco's Medium+fetal bovine serum, or RPMI-1640+fetal bovine serum) that contains components necessary for hematopoietic stem cells at approximately pH 7.0 to 7.3, in an incubator at 35°C to 38°C in a 5 to 7% CO₂ atmosphere for about 12 hours to about 72 hours, for example, for about 12 hours, about 24 hours, about 38 hours, about 48 hours, about 60 hours, about 72 hours, and the like.

For example, when the drug for genetic modification is applied in vivo, for example, to a living organism, for example, a whole animal including a human body, the method can be a therapeutic method. In that case, the bringing of the drug 1 for genetic modification containing the genome editing molecule 2 and the lipid nanoparticle 3 encapsulating the genome editing molecule 2 into contact with the target hematopoietic stem cell (S81) can be achieved by administration of the drug 1 for genetic modification to a living body or following the administration. The incubating of the hematopoietic stem cells in contact with the drug 1 for genetic modification (S82) may correspond to a period of time until the drug 1 for genetic modification or the genome editing molecule 2 no longer exhibits a desired function in vivo, for example, a period of time until the drug 1 for genetic modification or the genome editing molecule 2 is degraded or excreted.

That is, in this case, the target hematopoietic stem cell may be a hematopoietic stem cell in a state of being present in a living body. In this case, the contact between the drug 1 for genetic modification and the target hematopoietic stem cell is performed in vivo. In other words, when performed in vivo, the method can be understood as a method of treating a subject at least including bringing the drug 1 for genetic modification containing the genome editing molecule 2 and the lipid nanoparticle 3 encapsulating the genome editing molecule 2 into contact with the target hematopoietic stem cell (S81), or including S81 and S82 in need thereof.

When it is specified herein that the method is performed "in vitro", it is intended to exclude a method of treating an animal including a human performed in vivo. In addition, when it is specified in a "non-human living body (that is, an animal)" that the method is performed, it is intended to exclude a therapeutic method for a human performed in vivo. In addition, when "incubating" is specified as "culturing", it is intended to be maintained under specific conditions ex vivo.

As described above, according to the third arrangement, a technique for efficiently introducing a gene into a hematopoietic stem cell is provided. By the technique according to the third arrangement, it is possible to correct or normalize a genetic abnormality in the hematopoietic stem cells in vitro or in vivo by administering the drug for genetic modification according to the arrangement to a cell or a subject in need thereof.

### (Fourth Arrangement)

A further arrangement is a kit for genetic modification for a hematopoietic stem cell. Alternatively, the kit can also be understood as a kit for delivering a genome editing molecule 2 to a hematopoietic stem cell. In addition, the kit may be understood to be a kit for producing a drug 1 for genetic modification for a hematopoietic stem cell. The kit can contain any of the drugs 1 for genetic modification described above. For example, the kit may be provided in a state in which the drug 1 is immediately usable for a desired target hematopoietic stem cell. An example of a specific kit will be described with reference to FIG. 9. A kit 200 for genetic modification contains the drug 1 for genetic modification (FIG. 9).

The kit can be provided in a state of being accommodated in a container in an appropriate state and/or coexisting with an appropriate stabilizer, pH adjusting agent, buffering agent, and/or preservative so that the drug 1 for genetic modification is stably provided as a substance. Here, a genome editing molecule 2 may be provided as a mixture of a plurality of types of genome editing molecules as a genome editing molecule group. In addition, the kit may include an instruction for a user to appropriately use the drug 1 for genetic modification, for example, a manual related to addition of the drug.

According to the kit of the arrangement, a technique for efficiently introducing a gene into a hematopoietic stem cell is provided. For example, with the kit, the genome editing molecule 2 can be introduced into the hematopoietic stem cell, and the gene in the hematopoietic stem cell can be efficiently edited. In addition, by providing the kit, it is possible for a practitioner to use a technique for efficiently introducing a gene into a hematopoietic stem cell without being bound by time, technical, and/or economic restrictions. Therefore, it is possible or easy to administer the drug 1 for genetic modification according to the arrangement to a cell or a subject in need thereof, and it is possible to correct or normalize a genetic abnormality in the hematopoietic stem cells in vitro or in vivo.

### Examples

Hereinafter, arrangements will be further described based on results obtained from experiments performed as examples.

Experiment 1. Production of lipid nanoparticles encapsulating GFP gene

As the lipid nanoparticles encapsulating GFP gene, lipid nanoparticles were produced. This is specifically as follows. As a nucleic acid encapsulated in the lipid nanoparticle, messenger RNA of green fluorescent protein (GFP) gene was used. The nucleic acid was suspended in 10 mM HEPES (pH 7.3) to obtain a nucleic acid solution.

FFT-10, FFT-20, DOPE, DOTAP, cholesterol, and DMG-PEG were dissolved in ethanol at the charged lipid composition ratios (mol%) described in Table 1 to obtain lipid solutions of Examples 1 to 4.

### [Table 1]

**Table 1**

| | Charged lipid composition ratio (mol%) (FFT-10:FFT-20:DOPE:DOTAP: Cholesterol:DMG-PEG) |
|---|---|
| Example 1 | 2:30:2:17:45:4 |
| Example 2 | 0:32:5:9:51:3 |
| Example 3 | 15:30:9:4:38:4 |
| Example 4 | 0:20:6:23:48:3 |

The lipid solution and the nucleic acid solution were mixed using a microflow chip and a syringe pump. The resulting mixed solution was diluted 10 times with 10 mM HEPES (pH 7.3). Thereafter, the mixture was concentrated by ultrafiltration using an ultrafiltration filter (Amicon Ultra 0.5 Ultracel-50, manufactured by Merck) to obtain lipid nanoparticles of Examples 1 to 4, respectively.

Experiment 2. Measurement of concentration of encapsulated nucleic acid

The concentration of the nucleic acid encapsulated in each of the lipid nanoparticles of Examples 1 to 4 produced in Experiment 1 was measured using dsDNAQuantiFluor (registered trademark) RNA System (manufactured by Promega Corporation). The measurement was performed according to the manual attached to the kit.

### [Table 2]

**Table 2**

| | Encapsulated nucleic acid concentration (ng/µL) |
|---|---|
| Example 1 | 239 |
| Example 2 | 150 |
| Example 3 | 204 |
| Example 4 | 186 |

From the results shown in Table 2, it was confirmed that the messenger RNA of the GFP gene was encapsulated in a sufficient amount in any of the lipid nanoparticles of Examples 1 to 4 produced in Experiment 1.

### Experiment 3. Measurement of gene expression level

Cord blood-derived CD34 antigen-positive hematopoietic stem cells (CD34 positive cells) were suspended in a medium in which 50 ng/ml of SCF, 50 ng/ml of TPO, and 50 ng/ml of FLT3L were added to StemSpan. The resulting cells were seeded in a 96-well culture plate in an amount of 100 µL per well at the number of cells of 5 × 10⁴ cells. Subsequently, any lipid nanoparticles of the lipid nanoparticles of Examples 1 to 4 produced in Experiment 1 were added in an amount of 2 µL per well. Thereafter, the 96-well plate was accommodated in an incubator at 37°C in a 5% CO₂ atmosphere and allowed to stand, and cord blood-derived CD34 antigen-positive hematopoietic stem cells were cultured. After 48 hours of the addition of the lipid nanoparticles, the culture plate was taken out of the incubator, and the entire amount of the culture solution containing the cells was recovered. For the total amount of the recovered culture solution, the fluorescence intensity and the viable cell ratio of the GFP protein derived from the GFP gene were measured by a flow cytometer. The measurement was performed according to the manual attached to the flow cytometer.

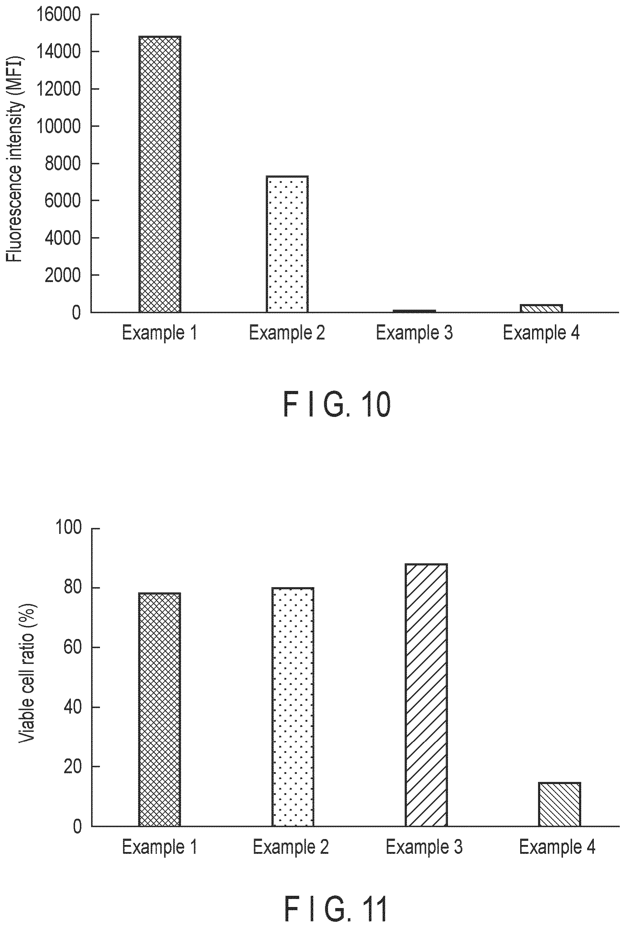

The results of Experiment 3 are illustrated in FIGS. 10 and 11. FIG. 10 illustrates the fluorescence intensity of the obtained GFP protein for the cells to which the lipid nanoparticles of Examples 1 to 4 were added, respectively. The vertical axis represents the fluorescence intensity of the GFP protein, and the horizontal axis represents the number of the corresponding lipid nanoparticles (Examples 1 to 4). In FIG. 11, the vertical axis represents the viable cell ratio, and the horizontal axis represents the number of the corresponding lipid nanoparticles (Examples 1 to 4).

As illustrated in FIG. 10, among the lipid nanoparticles of Examples 1 to 4 having different charged lipid composition ratios, in the CD34 positive cells to which the lipid nanoparticles of Example 1 were added and cultured, high fluorescence intensity exceeding MFI 10,000 was exhibited. On the other hand, for any of the CD34 positive cells to which the lipid nanoparticles of Examples 2 to 4 were added and cultured, the fluorescence intensity exceeding MFI 10,000 was not measured. As illustrated in FIG. 11, for any of the lipid nanoparticles, a large decrease in the viable cell ratio depending on the lipid composition ratio was not measured.

From the above results, it became clear that when the lipid nanoparticles of Example 1 were used among the lipid nanoparticles of Examples 1 to 4 used in the experiment, highly efficient delivery of nucleic acids to CD34 positive cells could be achieved while maintaining a high viable cell ratio.

### Experiment 4. Measurement of gene expression level

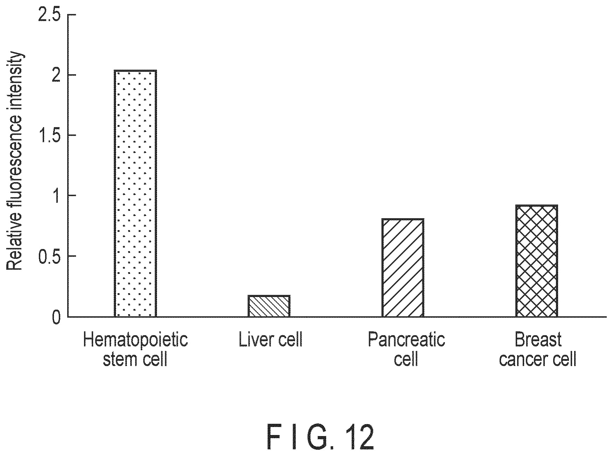

CD34 positive cells, liver cells, pancreatic cells, and breast cancer cells were seeded in a 96-well culture plate containing 100 µL of a medium in which cells were suspended per well so that the number of cells was 5 × 10⁴ cells. To each well, 2 µL of the lipid nanoparticles of either Example 1 or Example 2 were added. The culture plate thus prepared was accommodated in an incubator, allowed to stand, and cultured at 37°C in a 5% CO₂ atmosphere. After 48 hours of the addition of the lipid nanoparticles, the culture plate was taken out of the incubator, and the entire amount of the culture solution containing the cells was recovered. For the total amount of the recovered culture solution, the fluorescence intensity of the GFP protein derived from the GFP gene were measured by a flow cytometer. The measurement was performed according to the manual attached to the flow cytometer. The measurement results are illustrated in FIG. 12.

FIG. 12 illustrates the relative fluorescence intensity of the cultured cells obtained by adding the lipid nanoparticles of Example 1 when the fluorescence intensity obtained from the cells to which the lipid nanoparticles of Example 2 were added and cultured in each of the four types of cells is 1.

For the four types of CD34 positive cells such as hematopoietic stem cells, liver cells, pancreatic cells, and breast cancer cells, the relative fluorescence intensity decreased in the order of the hematopoietic stem cells, breast cancer cells, pancreatic cells, and liver cells (FIG. 12). The value of the relative fluorescence intensity was the largest in the hematopoietic stem cells, and the value exceeded the numerical value 2 only. On the other hand, the values of the relative fluorescence intensities in the other three types of cells were all less than 1.

From these results, it became clear that when the lipid composition of the lipid nanoparticles is set to be in the same range as the lipid composition of the lipid nanoparticles of Example 1, the efficiency of introducing a nucleic acid into a hematopoietic stem cell is improved as compared with liver cells, pancreatic cells, and breast cancer cells.

### Experiment 5. Production of lipid nanoparticles encapsulating genome editing gene

Lipid nanoparticles encapsulating a genome editing gene were produced. As the genome editing molecule encapsulated in the lipid nanoparticle, messenger RNA (Cas9 mRNA) of CRISPR-Cas9 gene and single-guide RNA (p53 sgRNA) targeting p53 gene were prepared. For these RNAs, two different types of nucleic acid modifications were used.

For Cas9 mRNA, mRNA without nucleic acid modification (SEQ ID NO: 5) and mRNA in which uridine was substituted with N1-methyl-pseudouridine (N1mΨU Cas9 mRNA) (SEQ ID NO: 6) were used. Hereinafter, Cas9 mRNA without nucleic acid modification is described as "mRNA-1", and Cas9 mRNA in which uridine is substituted with N1-methyl-pseudouridine is described as "mRNA-2". The base sequence of "mRNA-1" is described in Tables 3, and the base sequence of "mRNA-2" is described in Tables 4. In addition, among the two types of p53 sgRNAs used, one is referred to as "sgRNA-1", and the other is referred to as "sgRNA-2". The base sequence of "sgRNA-1" is described in Table 5, and the base sequence of "sgRNA-2" is described in Table 6.

### [Table 3]

**Table 3**

| | Base sequence |
|---|---|
| mRNA-1 | AGGAATAAACTAGTATTCTTCTGGTCCCCACAGACTCAGAGAGAACCCG CCACCatgGCCACCATGGACTATAAGGACCACGACGGAGACTACAAGGCA TCATGATATTGATTACAAAGACGATGACGATAAGATGGCCCCAAAGAAG AAGCGGAAGGTCGGTATCCACGGAGTCCCAGCAGCCGACAAGAAGTACA GCATCGGCCTGGACATCGGCACCAACTCTGTGGGCTGGGCCGTGATCAC CGACGAGTACAAGGTGCCCAGCAAGAAATTCAAGGTGCTGGGCAACACC GACCGGCACAGCATCAAGAAGAACCTGATCGGAGCCCTGCTGTTCGACA GCGGCGAAACAGCCGAGGCCACCCGGCTGAAGAGAACCGCCAGAAGAAG ATACACCAGACGGAAGAACCGGATCTGCTATCTGCAAGAGATCTTCAGC AACGAGATGGCCAAGGTGGACGACAGCTTCTTCCACAGACTGGAAGAGT CCTTCCTGGTGGAAGAGGATAAGAAGCACGAGCGGCACCCCATCTTCGG CAACATCGTGGACGAGGTGGCCTACCACGAGAAGTACCCCACCATCTAC CACCTGAGAAAGAAACTGGTGGACAGCACCGACAAGGCCGACCTGCGGC TGATCTATCTGGCCCTGGCCCACATGATCAAGTTCCGGGGCCACTTCCT GATCGAGGGCGACCTGAACCCCGACAACAGCGACGTGGACAAGCTGTTC ATCCAGCTGGTGCAGACCTACAACCAGCTGTTCGAGGAAAACCCCATCA ACGCCAGCGGCGTGGACGCCAAGGCCATCCTGTCTGCCAGACTGAGCAA GAGCAGACGGCTGGAAAATCTGATCGCCCAGCTGCCCGGCGAGAAGAAG AATGGCCTGTTCGGAAACCTGATTGCCCTGAGCCTGGGCCTGACCCCCA ACTTCAAGAGCAACTTCGACCTGGCCGAGGATGCCAAACTGCAGCTGAG CAAGGACACCTACGACGACGACCTGGACAACCTGCTGGCCCAGATCGGC GACCAGTACGCCGACCTGTTTCTGGCCGCCAAGAACCTGTCCGACGCCA TCCTGCTGAGCGACATCCTGAGAGTGAACACCGAGATCACCAAGGCCCC CCTGAGCGCCTCTATGATCAAGAGATACGACGAGCACCACCAGGACCTG ACCCTGCTGAAAGCTCTCGTGCGGCAGCAGCTGCCTGAGAAGTACAAAG AGATTTTCTTCGACCAGAGCAAGAACGGCTACGCCGGCTACATTGACGG CGGAGCCAGCCAGGAAGAGTTCTACAAGTTCATCAAGCCCATCCTGGAA AAGATGGACGGCACCGAGGAACTGCTCGTGAAGCTGAACAGAGAGGACC TGCTGCGGAAGCAGCGGACCTTCGACAACGGCAGCATCCCCCACCAGAT CCACCTGGGAGAGCTGCACGCCATTCTGCGGCGGCAGGAAGATTTTTAC CCATTCCTGAAGGACAACCGGGAAAAGATCGAGAAGATCCTGACCTTCC GCATCCCCTACTACGTGGGCCCTCTGGCCAGGGGAAACAGCAGATTCGC CTGGATGACCAGAAAGAGCGAGGAAACCATCACCCCCTGGAACTTCGAG GAAGTGGTGGACAAGGGCGCTTCCGCCCAGAGCTTCATCGAGCGGATGA CCAACTTCGATAAGAACCTGCCCAACGAGAAGGTGCTGCCCAAGCACAG CCTGCTGTACGAGTACTTCACCGTGTATAACGAGCTGACCAAAGTGAAA TACGTGACCGAGGGAATGAGAAAGCCCGCCTTCCTGAGCGGCGAGCAGA AAAAGGCCATCGTGGACCTGCTGTTCAAGACCAACCGGAAAGTGACCGT GAAGCAGCTGAAAGAGGACTACTTCAAGAAAATCGAGTGCTTCGACTCC GTGGAAATCTCCGGCGTGGAAGATCGGTTCAACGCCTCCCTGGGCACAT |
| mRNA-1 | ACCACGATCTGCTGAAAATTATCAAGGACAAGGACTTCCTGGACAATGA GGAAAACGAGGACATTCTGGAAGATATCGTGCTGACCCTGACACTGTTT GAGGACAGAGAGATGATCGAGGAACGGCTGAAAACCTATGCCCACCTGT TCGACGACAAAGTGATGAAGCAGCTGAAGCGGCGGAGATACACCGGCTG GGGCAGGCTGAGCCGGAAGCTGATCAACGGCATCCGGGACAAGCAGTCC GGCAAGACAATCCTGGATTTCCTGAAGTCCGACGGCTTCGCCAACAGAA ACTTCATGCAGCTGATCCACGACGACAGCCTGACCTTTAAAGAGGACAT CCAGAAAGCCCAGGTGTCCGGCCAGGGCGATAGCCTGCACGAGCACATT GCCAATCTGGCCGGCAGCCCCGCCATTAAGAAGGGCATCCTGCAGACAG TGAAGGTGGTGGACGAGCTCGTGAAAGTGATGGGCCGGCACAAGCCCGA GAACATCGTGATCGAAATGGCCAGAGAGAACCAGACCACCCAGAAGGGA CAGAAGAACAGCCGCGAGAGAATGAAGCGGATCGAAGAGGGCATCAAAG AGCTGGGCAGCCAGATCCTGAAAGAACACCCCGTGGAAAACACCCAGCT GCAGAACGAGAAGCTGTACCTGTACTACCTGCAGAATGGGCGGGATATG TACGTGGACCAGGAACTGGACATCAACCGGCTGTCCGACTACGATGTGG ACCATATCGTGCCTCAGAGCTTTCTGAAGGACGACTCCATCGACAACAA GGTGCTGACCAGAAGCGACAAGAACCGGGGCAAGAGCGACAACGTGCCC TCCGAAGAGGTCGTGAAGAAGATGAAGAACTACTGGCGGCAGCTGCTGA ACGCCAAGCTGATTACCCAGAGAAAGTTCGACAATCTGACCAAGGCCGA GAGAGGCGGCCTGAGCGAACTGGATAAGGCCGGCTTCATCAAGAGACAG CTGGTGGAAACCCGGCAGATCACAAAGCACGTGGCACAGATCCTGGACT CCCGGATGAACACTAAGTACGACGAGAATGACAAGCTGATCCGGGAAGT GAAAGTGATCACCCTGAAGTCCAAGCTGGTGTCCGATTTCCGGAAGGAT TTCCAGTTTTACAAAGTGCGCGAGATCAACAACTACCACCACGCCCACG ACGCCTACCTGAACGCCGTCGTGGGAACCGCCCTGATCAAAAAGTACCC TAAGCTGGAAAGCGAGTTCGTGTACGGCGACTACAAGGTGTACGACGTG CGGAAGATGATCGCCAAGAGCGAGCAGGAAATCGGCAAGGCTACCGCCA AGTACTTCTTCTACAGCAACATCATGAACTTTTTCAAGACCGAGATTAC CCTGGCCAACGGCGAGATCCGGAAGCGGCCTCTGATCGAGACAAACGGC GAAACCGGGGAGATCGTGTGGGATAAGGGCCGGGATTTTGCCACCGTGC GGAAAGTGCTGAGCATGCCCCAAGTGAATATCGTGAAAAAGACCGAGGT GCAGACAGGCGGCTTCAGCAAAGAGTCTATCCTGCCCAAGAGGAACAGC GATAAGCTGATCGCCAGAAAGAAGGACTGGGACCCTAAGAAGTACGGCG GCTTCGACAGCCCCACCGTGGCCTATTCTGTGCTGGTGGTGGCCAAAGT GGAAAAGGGCAAGTCCAAGAAACTGAAGAGTGTGAAAGAGCTGCTGGGG ATCACCATCATGGAAAGAAGCAGCTTCGAGAAGAATCCCATCGACTTTC TGGAAGCCAAGGGCTACAAAGAAGTGAAAAAGGACCTGATCATCAAGCT GCCTAAGTACTCCCTGTTCGAGCTGGAAAACGGCCGGAAGAGAATGCTG GCCTCTGCCGGCGAACTGCAGAAGGGAAACGAACTGGCCCTGCCCTCCA AATATGTGAACTTCCTGTACCTGGCCAGCCACTATGAGAAGCTGAAGGG |
| mRNA-1 | CTCCCCCGAGGATAATGAGCAGAAACAGCTGTTTGTGGAACAGCACAAG CACTACCTGGACGAGATCATCGAGCAGATCAGCGAGTTCTCCAAGAGAG TGATCCTGGCCGACGCTAATCTGGACAAAGTGCTGTCCGCCTACAACAA GCACCGGGATAAGCCCATCAGAGAGCAGGCCGAGAATATCATCCACCTG TTTACCCTGACCAATCTGGGAGCCCCTGCCGCCTTCAAGTACTTTGACA CCACCATCGACCGGAAGAGGTACACCAGCACCAAAGAGGTGCTGGACGC CACCCTGATCCACCAGAGCATCACCGGCCTGTACGAGACACGGATCGAC CTGTCTCAGCTGGGAGGCGACAAAAGGCCGGCGGCCACGAAAAAGGCCG GCCAGGCAAAAAAGAAAAAGtgaTGAGCTGGAGCCTCGGTGGCCTAGCT TCTTGCCCCTTGGGCCTCCCCCCAGCCCCTCCTCCCCTTCCTGCACCCG TACCCCCGTGGTCTTTGAATAAAGTCTGAGTGGGCGGC (SEQ ID NO: 5) |

### [Table 4]

**Table 4**

| | Base sequence |
|---|---|
| mRNA-2 | AGGAATAAACTAGTATTCTTCTGGTCCCCACAGACTCAGAGAGAACCCG CCACCGCCACCATGGACTATAAGGACCACGACGGAGACTACAAGGATCA TGATATTGATTACAAAGACGATGACGATAAGATGGCCCCAAAGAAGAAG CGGAAGGTCGGTATCCACGGAGTCCCAGCAGCCGACAAGAAGTACAGCA TCGGCCTGGACATCGGCACCAACTCTGTGGGCTGGGCCGTGATCACCGA CGAGTACAAGGTGCCCAGCAAGAAATTCAAGGTGCTGGGCAACACCGAC CGGCACAGCATCAAGAAGAACCTGATCGGAGCCCTGCTGTTCGACAGCG GCGAAACAGCCGAGGCCACCCGGCTGAAGAGAACCGCCAGAAGAAGATA CACCAGACGGAAGAACCGGATCTGCTATCTGCAAGAGATCTTCAGCAAC GAGATGGCCAAGGTGGACGACAGCTTCTTCCACAGACTGGAAGAGTCCT TCCTGGTGGAAGAGGATAAGAAGCACGAGCGGCACCCCATCTTCGGCAA CATCGTGGACGAGGTGGCCTACCACGAGAAGTACCCCACCATCTACCAC CTGAGAAAGAAACTGGTGGACAGCACCGACAAGGCCGACCTGCGGCTGA TCTATCTGGCCCTGGCCCACATGATCAAGTTCCGGGGCCACTTCCTGAT CGAGGGCGACCTGAACCCCGACAACAGCGACGTGGACAAGCTGTTCATC CAGCTGGTGCAGACCTACAACCAGCTGTTCGAGGAAAACCCCATCAACG CCAGCGGCGTGGACGCCAAGGCCATCCTGTCTGCCAGACTGAGCAAGAG CAGACGGCTGGAAAATCTGATCGCCCAGCTGCCCGGCGAGAAGAAGAAT GGCCTGTTCGGAAACCTGATTGCCCTGAGCCTGGGCCTGACCCCCAACT TCAAGAGCAACTTCGACCTGGCCGAGGATGCCAAACTGCAGCTGAGCAA GGACACCTACGACGACGACCTGGACAACCTGCTGGCCCAGATCGGCGAC CAGTACGCCGACCTGTTTCTGGCCGCCAAGAACCTGTCCGACGCCATCC TGCTGAGCGACATCCTGAGAGTGAACACCGAGATCACCAAGGCCCCCCT GAGCGCCTCTATGATCAAGAGATACGACGAGCACCACCAGGACCTGACC CTGCTGAAAGCTCTCGTGCGGCAGCAGCTGCCTGAGAAGTACAAAGAGA TTTTCTTCGACCAGAGCAAGAACGGCTACGCCGGCTACATTGACGGCGG AGCCAGCCAGGAAGAGTTCTACAAGTTCATCAAGCCCATCCTGGAAAAG ATGGACGGCACCGAGGAACTGCTCGTGAAGCTGAACAGAGAGGACCTGC TGCGGAAGCAGCGGACCTTCGACAACGGCAGCATCCCCCACCAGATCCA CCTGGGAGAGCTGCACGCCATTCTGCGGCGGCAGGAAGATTTTTACCCA TTCCTGAAGGACAACCGGGAAAAGATCGAGAAGATCCTGACCTTCCGCA TCCCCTACTACGTGGGCCCTCTGGCCAGGGGAAACAGCAGATTCGCCTG GATGACCAGAAAGAGCGAGGAAACCATCACCCCCTGGAACTTCGAGGAA GTGGTGGACAAGGGCGCTTCCGCCCAGAGCTTCATCGAGCGGATGACCA ACTTCGATAAGAACCTGCCCAACGAGAAGGTGCTGCCCAAGCACAGCCT GCTGTACGAGTACTTCACCGTGTATAACGAGCTGACCAAAGTGAAATAC GTGACCGAGGGAATGAGAAAGCCCGCCTTCCTGAGCGGCGAGCAGAAAA AGGCCATCGTGGACCTGCTGTTCAAGACCAACCGGAAAGTGACCGTGAA GCAGCTGAAAGAGGACTACTTCAAGAAAATCGAGTGCTTCGACTCCGTG GAAATCTCCGGCGTGGAAGATCGGTTCAACGCCTCCCTGGGCACATACC |
| mRNA-2 | ACGATCTGCTGAAAATTATCAAGGACAAGGACTTCCTGGACAATGAGGA AAACGAGGACATTCTGGAAGATATCGTGCTGACCCTGACACTGTTTGAG GACAGAGAGATGATCGAGGAACGGCTGAAAACCTATGCCCACCTGTTCG ACGACAAAGTGATGAAGCAGCTGAAGCGGCGGAGATACACCGGCTGGGG CAGGCTGAGCCGGAAGCTGATCAACGGCATCCGGGACAAGCAGTCCGGC AAGACAATCCTGGATTTCCTGAAGTCCGACGGCTTCGCCAACAGAAACT TCATGCAGCTGATCCACGACGACAGCCTGACCTTTAAAGAGGACATCCA GAAAGCCCAGGTGTCCGGCCAGGGCGATAGCCTGCACGAGCACATTGCC AATCTGGCCGGCAGCCCCGCCATTAAGAAGGGCATCCTGCAGACAGTGA AGGTGGTGGACGAGCTCGTGAAAGTGATGGGCCGGCACAAGCCCGAGAA CATCGTGATCGAAATGGCCAGAGAGAACCAGACCACCCAGAAGGGACAG AAGAACAGCCGCGAGAGAATGAAGCGGATCGAAGAGGGCATCAAAGAGC TGGGCAGCCAGATCCTGAAAGAACACCCCGTGGAAAACACCCAGCTGCA GAACGAGAAGCTGTACCTGTACTACCTGCAGAATGGGCGGGATATGTAC GTGGACCAGGAACTGGACATCAACCGGCTGTCCGACTACGATGTGGACC ATATCGTGCCTCAGAGCTTTCTGAAGGACGACTCCATCGACAACAAGGT GCTGACCAGAAGCGACAAGAACCGGGGCAAGAGCGACAACGTGCCCTCC GAAGAGGTCGTGAAGAAGATGAAGAACTACTGGCGGCAGCTGCTGAACG CCAAGCTGATTACCCAGAGAAAGTTCGACAATCTGACCAAGGCCGAGAG AGGCGGCCTGAGCGAACTGGATAAGGCCGGCTTCATCAAGAGACAGCTG GTGGAAACCCGGCAGATCACAAAGCACGTGGCACAGATCCTGGACTCCC GGATGAACACTAAGTACGACGAGAATGACAAGCTGATCCGGGAAGTGAA AGTGATCACCCTGAAGTCCAAGCTGGTGTCCGATTTCCGGAAGGATTTC CAGTTTTACAAAGTGCGCGAGATCAACAACTACCACCACGCCCACGACG CCTACCTGAACGCCGTCGTGGGAACCGCCCTGATCAAAAAGTACCCTAA GCTGGAAAGCGAGTTCGTGTACGGCGACTACAAGGTGTACGACGTGCGG AAGATGATCGCCAAGAGCGAGCAGGAAATCGGCAAGGCTACCGCCAAGT ACTTCTTCTACAGCAACATCATGAACTTTTTCAAGACCGAGATTACCCT GGCCAACGGCGAGATCCGGAAGCGGCCTCTGATCGAGACAAACGGCGAA ACCGGGGAGATCGTGTGGGATAAGGGCCGGGATTTTGCCACCGTGCGGA AAGTGCTGAGCATGCCCCAAGTGAATATCGTGAAAAAGACCGAGGTGCA GACAGGCGGCTTCAGCAAAGAGTCTATCCTGCCCAAGAGGAACAGCGAT AAGCTGATCGCCAGAAAGAAGGACTGGGACCCTAAGAAGTACGGCGGCT TCGACAGCCCCACCGTGGCCTATTCTGTGCTGGTGGTGGCCAAAGTGGA AAAGGGCAAGTCCAAGAAACTGAAGAGTGTGAAAGAGCTGCTGGGGATC ACCATCATGGAAAGAAGCAGCTTCGAGAAGAATCCCATCGACTTTCTGG AAGCCAAGGGCTACAAAGAAGTGAAAAAGGACCTGATCATCAAGCTGCC TAAGTACTCCCTGTTCGAGCTGGAAAACGGCCGGAAGAGAATGCTGGCC TCTGCCGGCGAACTGCAGAAGGGAAACGAACTGGCCCTGCCCTCCAAAT ATGTGAACTTCCTGTACCTGGCCAGCCACTATGAGAAGCTGAAGGGCTC |
| mRNA-2 | CCCCGAGGATAATGAGCAGAAACAGCTGTTTGTGGAACAGCACAAGCAC TACCTGGACGAGATCATCGAGCAGATCAGCGAGTTCTCCAAGAGAGTGA TCCTGGCCGACGCTAATCTGGACAAAGTGCTGTCCGCCTACAACAAGCA CCGGGATAAGCCCATCAGAGAGCAGGCCGAGAATATCATCCACCTGTTT ACCCTGACCAATCTGGGAGCCCCTGCCGCCTTCAAGTACTTTGACACCA CCATCGACCGGAAGAGGTACACCAGCACCAAAGAGGTGCTGGACGCCAC CCTGATCCACCAGAGCATCACCGGCCTGTACGAGACACGGATCGACCTG TCTCAGCTGGGAGGCGACAAAAGGCCGGCGGCCACGAAAAAGGCCGGCC AGGCAAAAAAGAAAAAGGGCGGTGAGGGCAGAGGAAGTCTGCTAACATG CGGTGACGTCGAGGAGAATCCTGGCCCAATGGTGAGCAAGGGCGAGGAG GATAACATGGCCATCATCAAGGAGTTCATGCGCTTCAAGGTGCACATGG AGGGCTCCGTGAACGGCCACGAGTTCGAGATCGAGGGCGAGGGCGAGGG CCGCCCCTACGAGGGCACCCAGACCGCCAAGCTGAAGGTGACCAAGGGT GGCCCCCTGCCCTTCGCCTGGGACATCCTGTCCCCTCAGTTCATGTACG GCTCCAAGGCCTACGTGAAGCACCCCGCCGACATCCCCGACTACTTGAA GCTGTCCTTCCCCGAGGGCTTCAAGTGGGAGCGCGTGATGAACTTCGAG GACGGCGGCGTGGTGACCGTGACCCAGGACTCCTCCCTGCAGGACGGCG AGTTCATCTACAAGGTGAAGCTGCGCGGCACCAACTTCCCCTCCGACGG CCCCGTAATGCAGAAGAAGACCATGGGCTGGGAGGCCTCCTCCGAGCGG ATGTACCCCGAGGACGGCGCCCTGAAGGGCGAGATCAAGCAGAGGCTGA AGCTGAAGGACGGCGGCCACTACGACGCTGAGGTCAAGACCACCTACAA GGCCAAGAAGCCCGTGCAGCTGCCCGGCGCCTACAACGTCAACATCAAG TTGGACATCACCTCCCACAACGAGGACTACACCATCGTGGAACAGTACG AACGCGCCGAGGGCCGCCACTCCACCGGCGGCATGGACGAGCTGTACAA GTAGTGAGCTGGAGCCTCGGTGGCCTAGCTTCTTGCCCCTTGGGCCTCC CCCCAGCCCCTCCTCCCCTTCCTGCACCCGTACCCCCGTGGTCTTTGAA TAAAGTCTGAGTGGGCGGC (SEQ ID NO: 6) |

### [Table 5]

**Table 5**

| | Base sequence |
|---|---|
| sgRNA-1 | CGCUAUCUGAGCAGCGCUCA (SEQ ID NO: 7) |

### [Table 6]

**Table 6**

| | Base sequence |
|---|---|
| sgRNA-2 | CUCGGAUAAGAUGCUGAGGA (SEQ ID NO: 8)) |

These mRNA and sgRNA were mixed in the combinations shown in Table 7, and the mixed nucleic acid was suspended in 10 mM HEPES (pH 7.3) to obtain a nucleic acid solution. On the other hand, a lipid nanoparticle material having a lipid composition equal to that of the lipid nanoparticle of Example 1 produced in Experiment 1 was dissolved in ethanol to obtain a lipid solution. The lipid solution and the nucleic acid solution were mixed using a microflow chip and a syringe pump. The mixed solution was diluted 10 times with 10 mM HEPES (pH 7.3), and then concentrated with an ultrafiltration filter (Amicon Ultra 0.5 Ultracel-50, manufactured by Merck) to obtain lipid nanoparticles. These lipid nanoparticles were used as Examples 5 to 8, respectively (Table 7).

### [Table 7]

**Table 7**

| | Cas9 mRNA | p53 sgRNA |
|---|---|---|
| Example 5 | mRNA-1 | sgRNA-1 |
| Example 6 | mRNA-1 | sgRNA-2 |
| Example 7 | mRNA-2 | sgRNA-1 |
| Example 8 | mRNA-2 | sgRNA-2 |

### Experiment 6. Evaluation of gene mutation by genome editing

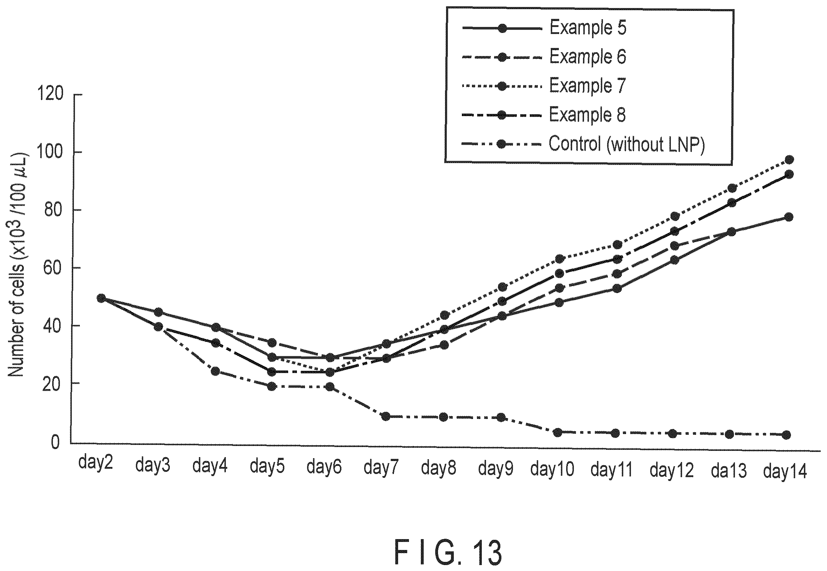

By adding 100 µL of the cell suspension in the medium to each well of the 96-well culture plate, CD34 positive cells were seeded so that the number of cells was 5 × 10⁴ cells per well. 2 µL of the lipid nanoparticles of Examples 5 to 8 were added to each well. Thereafter, the culture plate was accommodated in an incubator and cultured at 37°C in a 5% CO₂ atmosphere. After 48 hours of culture, the number of cells was measured, and the cells were re-seeded so as to be 5 × 10⁴ cells in the medium per well. A p53-MDM2 interaction inhibitor (750 nM) was added thereto, and the cells were cultured at 37°C in a 5% CO₂ atmosphere. For 12 days after the addition of the p53-MDM2 interaction inhibitor, samples were sorted every 24 hours to measure the number of cells, and the presence or absence of p53 gene mutation was evaluated. The results are shown in the graph of FIG. 13. In FIG. 13, the vertical axis represents the number of cells, and the horizontal axis represents the number of elapsed days.

From FIG. 13, in the cells to which the lipid nanoparticles of Examples 5 to 8 were added, respectively, the cell proliferation was activated after Day 7 of culture, and the number of cells increased. On Day 14 of culture, the number of cells was 1.5 times or more higher than that on Day 2. Regardless of the presence or absence of nucleic acid modification of Cas9 mRNA encapsulated in the lipid nanoparticles of Examples 5 to 8 and the difference in the sequence of p53 sgRNA, the timing of activation of cell proliferation and the rate of increase in the number of cells were equivalent. The cells to which the lipid nanoparticles were not added showed the same number of cells as the cells to which the lipid nanoparticles were added until Day 6 of culture. However, cell proliferation did not occur after Day 7 of culture, and most cells were killed by Day 14 of culture.

DNA was extracted from each sample cultured for 14 days, and genome analysis was performed by the Sanger sequencing method to evaluate a rate of base insertion and base deletion of p53 gene (Indel rate). The Indel rates calculated by genome analysis are shown in Table 8.

### [Table 8]

**Table 8**

| | Indel rate |
|---|---|
| Example 5 | 32% |
| Example 6 | 41% |
| Example 7 | 28% |
| Example 8 | 43% |

As shown in Table 8, in all of the cells cultured for 14 days to which the lipid nanoparticles of Examples 5 to 8 were added, the mutation of the p53 gene due to the occurrence of insertion or deletion in the base sequence was confirmed. In addition, the lipid nanoparticles of Examples 6 and 8 encapsulating sgRNA-2 exhibited a higher Indel rate than those of the lipid nanoparticles of Examples 5 and 7 encapsulating sgRNA-1. Note that the presence or absence of nucleic acid modification of Cas9 mRNA did not affect the Indel rate.

From the above results, it became clear that by making the lipid composition of the lipid nanoparticle equivalent to that of the lipid nanoparticle of Example 1, Cas9 mRNA and sgRNA can be efficiently introduced into CD34 positive cells, and a mutation can be introduced into a target gene to deactivate the gene.

As described above, it was confirmed that a technique for editing a gene in a hematopoietic stem cell is provided by the lipid nanoparticle, the delivery method, and the kit according to the arrangement.
While certain arrangements have been described, these arrangements have been presented by way of example only, and are not intended to limit the scope of the claims. Indeed, the drug described herein may be embodied in a variety of other forms; furthermore, various omissions, substitutions and changes in the form of the drug described herein may be made.

### Clause 1

A drug(1) for genetic modification of a hematopoietic stem cell characterized by comprising:
a genome editing molecule(2) for performing genome editing on a hematopoietic stem cell, and a lipid nanoparticle(3) encapsulating the genome editing molecule,
the lipid nanoparticle including a lipid membrane(30) having a lumen,
the lipid membrane having a lipid composition containing at least a first lipid of the following Formula I and a second lipid of the following Formula II,
the second lipid being contained in a larger amount than the first lipid, and a total of the first lipid and the second lipid being 40 mol% or less, and
a cationic lipid contained in the lipid composition being 60 mol% or less.

### Clause 2

The drug according to Clause 1, characterized in that the total of the first lipid and the second lipid is 30 to 40 mol% in the lipid composition.

### Clause 3

The drug according to Clause 1, characterized in that the first lipid is 10 mol% or less in the lipid composition.

### Clause 4

The drug according to Clause 1, characterized in that the lipid composition further contains a third lipid as a cationic lipid other than the first lipid and the second lipid, a neutral lipid, cholesterol, and a PEG-modified lipid, and a total of these components is 70 mol% or less.

### Clause 5

The drug according to Clause 4, characterized in that the third lipid is contained in the lipid composition in a larger amount than the neutral lipid.

### Clause 6

The drug according to Clause 5, characterized in that the lipid composition contains the first lipid, the second lipid, the neutral lipid, the third lipid, the cholesterol, and the PEG-modified lipid, the components of the lipid composition are in a range of, as mol%, first lipid : second lipid : neutral lipid : third lipid : cholesterol : PEG-modified lipid = 1 to 3:29 to 31:1 to 3:16 to 18:44 to 46:3 to 5, and a value is selected so that a total of the components is 100 mol%.

### Clause 7

The drug according to Clause 6, characterized in that the neutral lipid is 1,2-dioleoyl-sn-glycero-3-phosphoethanolamine (DOPE), and the third lipid is 1,2-dioleoyl-3-trimethylammonium-propane (DOTAP).

### Clause 8

The drug according to Clause 6, characterized in that the PEG-modified lipid has a maleimide-modified functional group.

### Clause 9

The drug according to Clause 1, characterized in that the genome editing molecule contains Cas protein and/or guide RNA.

### Clause 10

The drug according to Clause 9, characterized in that the Cas protein is CRISPR-Cas9.

### Clause 11

The drug according to Clause 9, characterized in that the genome editing molecule contains a nucleic acid encoding the Cas protein and/or a nucleic acid encoding the guide RNA.

### Clause 12

The drug according to Clause 1, characterized in that a plurality of types of the genome editing molecules different from each other are encapsulated in a lipid nanoparticle, or a plurality of types of the genome editing molecules different from each other are divided and encapsulated in a plurality of lipid nanoparticles.

### Clause 13

A method of delivering a genome editing molecule to a hematopoietic stem cell, the method characterized by comprising:
bringing the drug for genetic modification according to Clause 1 into contact with a target hematopoietic stem cell in vitro; and
incubating the hematopoietic stem cell in contact with the drug for genetic modification.

### Clause 14

A method of modifying a gene in a hematopoietic stem cell, the method characterized by comprising:
bringing the drug for genetic modification according to Clause 1 into contact with a target hematopoietic stem cell in vitro; and
incubating the hematopoietic stem cell in contact with the drug for genetic modification.

### Clause 15

A drug delivery carrier characterized by comprising:
a lipid nanoparticle including a lipid membrane having a lumen, the drug delivery carrier being used for delivering a substance encapsulated in the lumen to a hematopoietic stem cell,
the lipid membrane having a lipid composition containing at least a first lipid of the following Formula I and a second lipid of the following Formula II,
the second lipid being contained in a larger amount than the first lipid, and a total of the first lipid and the second lipid being 40 mol% or less, and
a cationic lipid contained in the lipid composition being 60 mol% or less.

## Claims

1. A drug(1) for genetic modification of a hematopoietic stem cell **characterized by** comprising:
a genome editing molecule(2) for performing genome editing on a hematopoietic stem cell, and a lipid nanoparticle(3) encapsulating the genome editing molecule,
the lipid nanoparticle including a lipid membrane(30) having a lumen,
the lipid membrane having a lipid composition containing at least a first lipid of the following Formula I and a second lipid of the following Formula II, 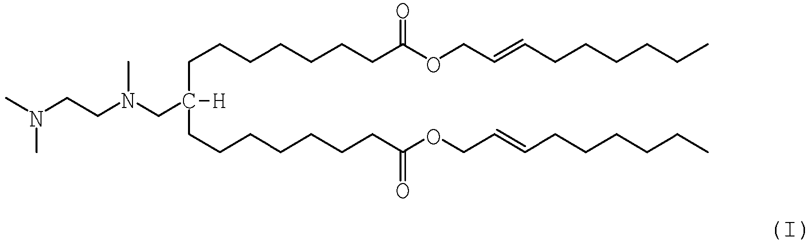 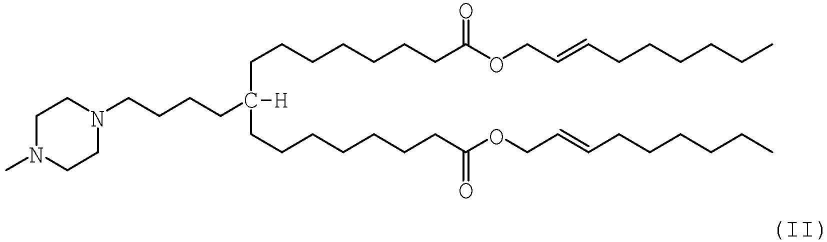
the second lipid being contained in a larger amount than the first lipid, and a total of the first lipid and the second lipid being 40 mol% or less, and
a cationic lipid contained in the lipid composition being 60 mol% or less.

2. The drug according to claim 1, wherein the total of the first lipid and the second lipid is 30 to 40 mol% in the lipid composition.

3. The drug according to claim 1, **characterized in that** the first lipid is 10 mol% or less in the lipid composition.

4. The drug according to claim 1, **characterized in that** the lipid composition further contains a third lipid as a cationic lipid other than the first lipid and the second lipid, a neutral lipid, cholesterol, and a PEG-modified lipid, and a total of these components is 70 mol% or less.

5. The drug according to claim 4, **characterized in that** the third lipid is contained in the lipid composition in a larger amount than the neutral lipid.

6. The drug according to claim 5, **characterized in that** the lipid composition contains the first lipid, the second lipid, the neutral lipid, the third lipid, the cholesterol, and the PEG-modified lipid, the components of the lipid composition are in a range of, as mol%, first lipid : second lipid : neutral lipid : third lipid : cholesterol : PEG-modified lipid = 1 to 3:29 to 31:1 to 3:16 to 18:44 to 46:3 to 5, and a value is selected so that a total of the components is 100 mol%.

7. The drug according to claim 6, **characterized in that** the neutral lipid is 1,2-dioleoyl-sn-glycero-3-phosphoethanolamine (DOPE), and the third lipid is 1,2-dioleoyl-3-trimethylammonium-propane (DOTAP).

8. The drug according to claim 6, **characterized in that** the PEG-modified lipid has a maleimide-modified functional group.

9. The drug according to claim 1, **characterized in that** the genome editing molecule contains Cas protein and/or guide RNA.

10. The drug according to claim 9, **characterized in that** the Cas protein is CRISPR-Cas9.

11. The drug according to claim 9, **characterized in that** the genome editing molecule contains a nucleic acid encoding the Cas protein and/or a nucleic acid encoding the guide RNA.

12. The drug according to claim 1, **characterized in that** a plurality of types of the genome editing molecules different from each other are encapsulated in a lipid nanoparticle, or a plurality of types of the genome editing molecules different from each other are divided and encapsulated in a plurality of lipid nanoparticles.

13. A method of delivering a genome editing molecule to a hematopoietic stem cell, the method **characterized by** comprising:
bringing the drug for genetic modification according to claim 1 into contact with a target hematopoietic stem cell in vitro; and
incubating the hematopoietic stem cell in contact with the drug for genetic modification.

14. A method of modifying a gene in a hematopoietic stem cell, the method **characterized by** comprising:
bringing the drug for genetic modification according to claim 1 into contact with a target hematopoietic stem cell in vitro; and
incubating the hematopoietic stem cell in contact with the drug for genetic modification.

15. A drug delivery carrier **characterized by** comprising:
a lipid nanoparticle including a lipid membrane having a lumen, the drug delivery carrier being used for delivering a substance encapsulated in the lumen to a hematopoietic stem cell,
the lipid membrane having a lipid composition containing at least a first lipid of the following Formula I and a second lipid of the following Formula II, 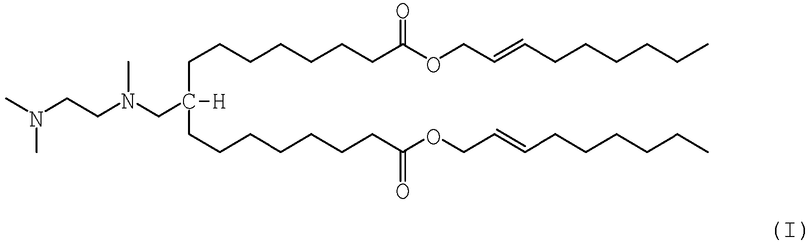 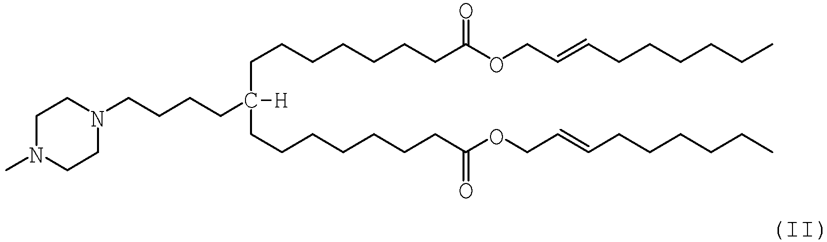
the second lipid being contained in a larger amount than the first lipid, and a total of the first lipid and the second lipid being 40 mol% or less, and
a cationic lipid contained in the lipid composition being 60 mol% or less.
